# EUROPEAN PATENT SPECIFICATION

(11) **EP 0 967 927 B1**
(45) Date of publication and mention of the grant of the patent: **16.04.2003**
(21) Application number: 98903376.6
(22) Date of filing: 08.01.1998
(51) Int. Cl.: A61B 19/00, A61B 5/06, A61F 13/44

(54) **SURGICAL IMPLEMENT DETECTOR UTILIZING A SMART MARKER**
SUCHGERÄT FÜR CHIRURGISCHE INSTRUMENTE VERSEHEN MIT MARKIERUNGEN
DETECTEUR D'INSTRUMENT CHIRURGICAL UTILISANT UN MARQUEUR INTELLIGENT

(30) Priority: 08.01.1997 US 780697
(43) Date of publication of application: 05.01.2000
(60) Divisional application: 02011242.1
(73) Proprietor: FABIAN, Carl E., Miami Shores, FL 33138 (US); ANDERSON, Philip M., Madison, NJ 07940 (US)
(72) Inventor: FABIAN, Carl E., Miami Shores, FL 33138 (US); ANDERSON, Philip M., Madison, NJ 07940 (US)
(74) Representative: Price, Nigel John King
(86) International application number: US9800145
(87) International publication number: WO98030166

(56) References cited:
- WO-A-94/17767
- FR-A- 2 580 172
- GB-A- 1 274 061
- US-A- 5 057 095
- US-A- 5 456 718

## Description

The present invention relates to an apparatus and a method for detecting a marked surgical implement such as a sponge, clamp, or catheter within a surgical wound in human or animal tissue irrespective of its position or orientation therewithin.

During the course of a surgical operation it is frequently necessary for articles, such as surgical sponges, gauzes, instruments, needles, and the like, to be placed into a wound cavity. Notwithstanding rigorous precautions attendant surgical procedures, such items are sometimes inadvertently lost during surgery and remain within the wound. When this happens the patient can encounter serious consequences, including pain, infection, intestinal obstruction, and even death. The problem of retained surgical implements has existed since the earliest days of surgery. Procedures conventionally employed to prevent post-surgical implement retention include a manual search of the wound cavity by the surgeon prior to closure and a careful accounting for all materials inserted and removed from the wound. The accounting function is customarily carried out by the operating room staff, usually the circulating nurse. Despite these precautionary measures the accidental retention of surgical implements continues to occur with disturbing regularity, even in prestigious institutions, and is regarded by surgeons as a major unsolved problem.

At present, manual search and physical count remain the primary methods used to prevent retention of surgical implements. Most surgical instruments are composed of metal, and are easily detectable by x-ray. Sponges are usually marked with radiopaque markers to make them additionally visible by x-ray. But x-rays are not routinely carried out upon completion of the operation because of several disadvantages, including inconvenience, expense, loss of operative time, and radiation exposure. Performing an x-ray after the patient has left the operating room suffers from some of the same disadvantages. Moreover, even when postoperative x-rays arc taken, retained surgical items are frequently overlooked, and even if detected at that time, require a second operation to effect their removal.

To overcome the difficulty of detecting retained surgical implements, it has been suggested that the implements be provided with a radioactive tracer. This technique, disclosed by U.S. Patent 2,740,405 to Riordan, is subject to obvious hazards associated with use, storage and disposal of radioactive materials.

It has also been proposed that surgical sponges be marked with a flexible plastic impregnated with either paramagnetic or ferromagnetic materials in the form of powders. Detection of these marked sponges is accomplished by a metal detector. This method, taught by U. S. Patent 3,422,816 to Robinson et al., provides very small signals difficult to detect over the width of a patient's body. In addition, the Robinson et al. technique provides no discrimination against other metal objects, such as staples which, though present within the surgical wound, are appointed for retention therewithin.

Yet another proposal, advanced by U.S. Patent 3,587,583 to Grcenberg, involves use of surgical sponges marked with magnetized particles whose presence is detectable with magnetodiodes. In practice, however, the magnetic field generated by these particles is too small to be readily detected by the diodes.

U. S. Patent 4,114,601 to Abels discloses the use of a small transponder fixed to a surgical sponge or instrument. This transponder exhibits gyromagnetic resonance at preselected frequencies. Detection is accomplished by nonlinear mixing of two frequencies impinging upon the transponder. The gyromagnetic resonance effect disclosed by Abels is a high frequency phenomenon, existing at frequencies of the order of about 5 gigahertz (5,000,000,000 cycles/sec). These frequencies, known as microwaves, are absorbed readily by animal tissue and are, in fact, used in microwave ovens for cooking. In use of the Abels type transponder, the energy developed goes primarily into heating tissue, rather than exciting the transponder into gyromagnetic resonance.

British Patent No. 2.242.551 discloses a surgical implement having a bar code attached thereto. The bar code is optically read and oftentimes becomes obscured by blood and tissue fragments within the wound cavity. Hence its use is generally impractical for surgical procedures.

U. S. Patent 5,057,095 to Fabian and Anderson discloses the use of a resonant transponder fixed to a surgical sponge or instrument, and U. S. Patent 5,190,059 to Fabian and Anderson discloses a powered marker, while U. S. Patent 5,188,126 to Fabian and Anderson discloses a capacitive marker. These patents do not provide for storing a code in a marker.

FR-A1-2 580 172 discloses an apparatus in accordance with the pre-characterising section of claim 1.

According to a first aspect of the present invention there is provided an apparatus for detecting a surgical implement in human or animal tissue comprising:
field-generating means for generating an electromagnetic field having a predetermined frequency;
a marker secured to a surgical implement appointed for disposition within said tissue; and
detecting means;
characterised in that:
said marker has a memory for storing a predetermined code and is arranged to be powered by said electromagnetic field to become operative in the presence of said electromagnetic field to transmit said predetermined code;
said predetermined frequency is in the range of 5 kHz to 3 MHz; and
said detecting means is arranged to receive, decode and verify said predetermined code, and correlate it with said surgical instrument, whereby to enable said surgical instrument to be uniquely identified without the need to physically connect said marker to either said field-generating means or said detecting means.

As explained hereinafter, in the illustrated embodiments the read code is added to a code list for inserted implements. Upon withdrawal of the implement from the surgical wound, another reading is taken and matched by a microprocessor against the code list. Implements not accounted for are positively identified prior to closure of the wound. The reader can be used at any time to probe the surgical wound for markers and attached implements.

According to a second aspect of the present invention there is provided a method for detecting a surgical implement in human or animal tissue comprising the steps of:
generating an electromagnetic field, having a predetermined frequency in the range of 5 kHz to 3 MHz, in the vicinity of said tissue;
attaching a marker to a surgical instrument appointed for disposition within said tissue, said marker having a memory storing a predetermined code;
powering said marker by placing said marker in the presence of said electromagnetic field;
transmitting said predetermined code; and
detecting said transmitted code and correlating it with said surgical implement, thereby enabling said surgical instrument to be uniquely identified.

As will become apparent from the following description of the illustrated preferred embodiments, the preferred embodiments of apparatus and method detect retained surgical implements with far greater accuracy and efficiency than methods and means involving a physical count of implements that enter and exit the wound. Coding of the marker uniquely differentiates it from other markers, assuring positive identification and specification of the implement to which it is attached. The apparatus is inexpensive to construct, more efficient and reliable than postoperative X-rays and avoids risk to the environment and personnel posed by radioactive tracers. The signal is more easily distinguished than signals generated by magnetic detection systems, and is generated without the heating of tissue caused by microwave detection systems.

The invention will be more fully understood and further advantages will become apparent when reference is made to the following detailed description of the preferred embodiment of the invention and the accompanying drawings in which:
Fig. 1 is a block diagram of a surgical implement detector reader and marker incorporating the elements of the present invention;
Fig. 2 is a schematic diagram depicting an alternate antenna configuration for the detector of Fig. 1;
Fig. 3 is a circuit diagram of a marker suited for use in the detector of Fig. 1; and
Fig. 4 is a block diagram showing an alternate embodiment of a surgical implement detector reader;

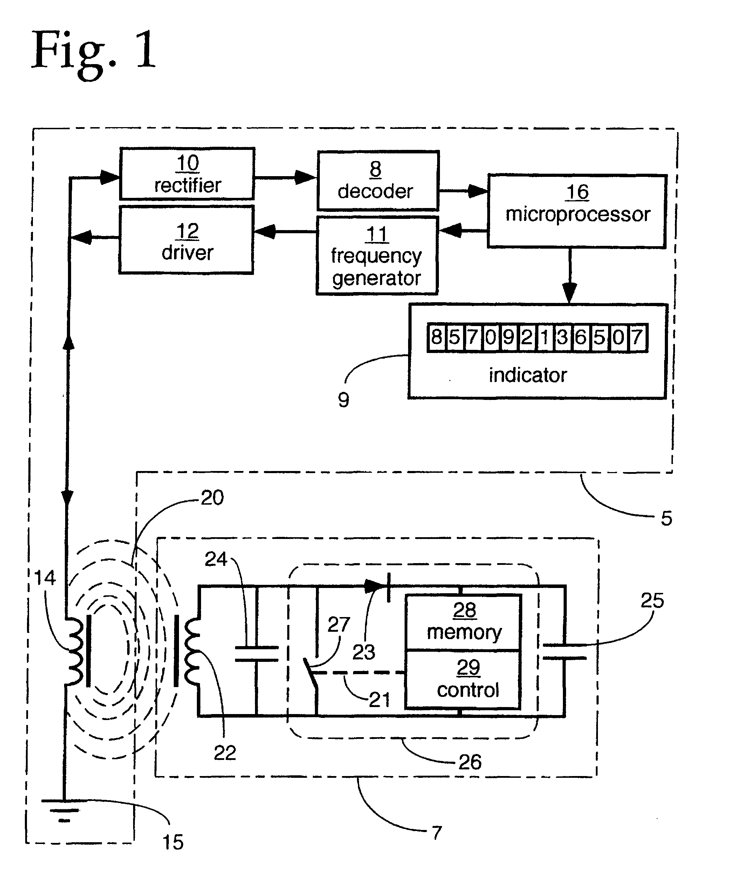

Referring to the drawings, there is shown in Fig. 1 an apparatus for detecting a surgical implement in human or animal tissue Frequency generator 11, driver 12, and reader antenna 14 provide means for generating magnetic field 20 at a predetermined frequency. Rectifier 10, provide a means for receiving the predetermined code of marker 7, and decoder 8 provides a means for decoding the predetermined code. Microprocessor 16 provides the means for verifying the predetermined code. Indicator 9 provides a means for indicating reception of the predetermined code. Alternatively, verification of the predetermined code can be performed manually.

In Fig. 1, reader 5 comprises microprocessor 16, frequency generator 11, driver 12, rectifier 10, decoder 8, indicator 9, and reader antenna 14. The reader is shown by the dashed box labeled 5 in Fig. 1. Microprocessor 16 directs frequency generator 11 to provide a predetermined frequency to driver 12. Preferably, the predetermined frequency is between 50 kHz and 300 kHz. Driver 12 in turn supplies reader antenna 14 with a current of the predetermined frequency, thus in turn, generating magnetic field 20 having the predetermined frequency. Preferably, reader antenna 14 is comprised of a coil with a ferrite core.

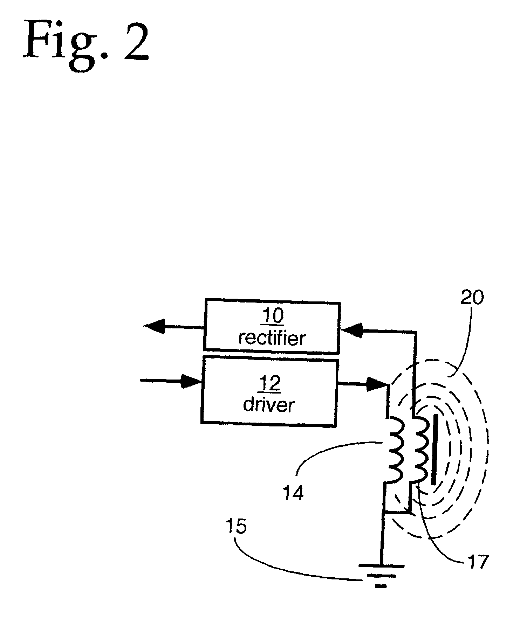

Marker 7 comprises marker antenna 22, tuning capacitor 24, diode 23, capacitor 25, memory 28, control 29, and switch 27. The marker is indicated by the dashed box labeled 7 in Fig. 1. Preferably, memory 28 is an EEPROM provided with a predetermined code. Marker antenna 22 is comprised of a coil and ferrite core having dimensions preferably near 6 by 30 mm. Tuning capacitor 24 forms, with marker antenna 22, a tuned tank circuit having a resonant frequency equal to the predetermined frequency of magnetic field 20. When marker 7 is sufficiently close to reader antenna 14, magnetic field 20 will generate a voltage in marker antenna 22 that is rectified by diode 23 causing capacitor 25 to charge. When capacitor 25 reaches a predetermined voltage, memory 28, and control 29 become operational. The predetermined voltage is typically between 3 and 5 volts, depending on the choice of marker components. Control 29, via connection 21, causes switch 27 to open and close in sequence according to the predetermined code of memory 28. The opening and closing of switch 27 causes marker antenna 22 to vary impedance. This varying impedance disturbs magnetic field 20, which in turn disturbs the voltage from driver 12 in reader antenna 14. The varying voltage is rectified by rectifier 10. Decoder 8 filters and demodulates the voltage to reconstruct the predetermined code of memory 28. Microprocessor 16 determines validity of the code by comparing the code with a programmed internal code list. If a match is found, indicator 9 is activated. Preferably, decoder 8 and frequency generator 11 are combined as an integrated circuit of the type m3394b. The m3394b requires a quartz crystal and a maximum of 2 RC support circuits. Alternatively as shown in Fig. 2, reader antenna 14 comprises a second coil 17 connected to rectifier 10 for sensing the disturbance of magnetic field 20. Circuit ground is indicated by 15 in Fig. 1.

Preferably as shown in Fig. 1, diode 23, memory 28, control 29, and switch 27 are combined together into a single integrated circuit 26 in marker 7. The integrated circuit is indicated by the dashed box labeled 26 in Fig. 1.

For marker antenna 22 to be effective, the predetermined frequency of magnetic field 20 should be below 3 megahertz and above 5 kilohertz. Preferably, the predetermined frequency of magnetic field 20 ranges from about 50 to 300 kilohertz. It is even more preferable that the predetermined frequency of magnetic field 20 be near or at 125 kilohertz.

The method for detecting a surgical implement in human or animal tissue comprises the steps of: generating a magnetic field having a predetermined frequency; attaching a marker to a surgical implement appointed for disposition within the tissue, the marker being powered by the magnetic field and being operative in its presence to transmit a predetermined code; receiving the predetermined code; decoding the predetermined code; verifying the predetermined code; and indicating receipt of the predetermined code.

In practice, a marker is attached to a surgical implement and the reader is used to read and record the marker's predetermined code to a inventory list in the microprocessor. On removal of a marker from the surgical wound, the reader is again used to read and record the predetermined code, which is matched with the inventory list. Unmatched codes indicate unaccounted for implements. The user is alerted by the mismatch, and a search of the wound with the reader is made to locate any missing implements. Prior to closure of the surgical wound, the reader is placed near the wound to perform a fail-safe search for implements.

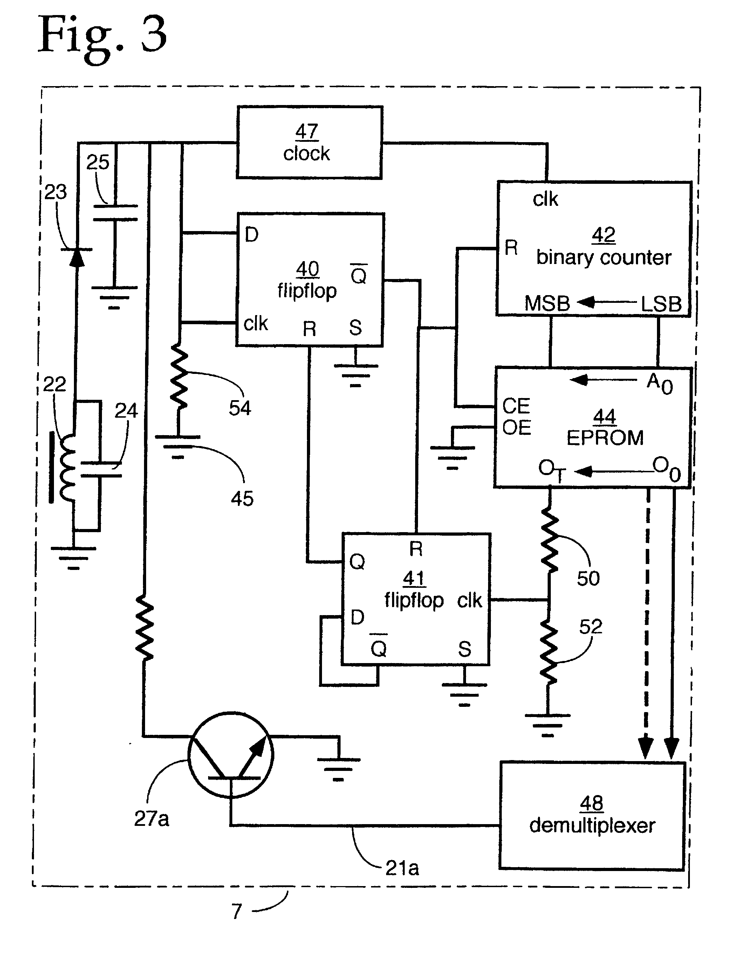

Figure 3 shows one possible circuit for marker 7. A voltage induced in marker antenna 22 and tuning capacitor 24, and rectified by diode 23 charges capacitor 25. Capacitor 25 is now the source of power for the other elements of marker 7. Clock 47 provides circuit timing. Flip-flop 40 causing initialization of binary counter 42 and EEPROM 44. With each clock pulse from clock 47, binary counter 42 counts up from zero to one plus the number of digits of the predetermined code. With each count, EEPROM 44 outputs the corresponding digit of the predetermined code to demultiplexer 48. The last digit in memory of EEPROM 44 provides an end-of-sequence pulse to flip-flop 41, which in turn provides a reset pulse to binary counter 42 and EEPROM 44. Demultiplexer 48 sends each digit of the predetermined code along control line 21a to switch 27a. In this manner, marker antenna 22 is either shorted or left unaffected depending on the digit of the predetermined code considered. Resistors 54, 50, and 52 provide reference voltages with respect to ground 45.

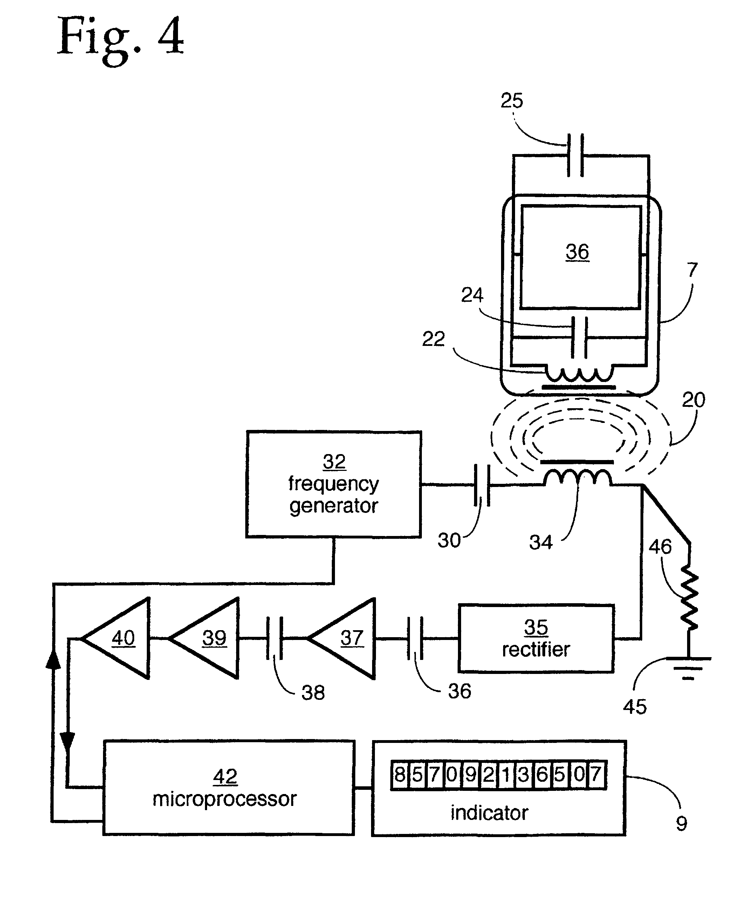

Fig. 4 shows an alternate construction of reader 5. Frequency generator 32 is coupled to antenna 34 through capacitor 30 creating magnetic field 20. Marker 7 disturbs magnetic field 20 as described above. The disturbed voltage is sensed and rectified by rectifier 35. Tuned amplifiers 37 and 39 along with coupling capacitors 36 and 38 filter and demodulate the sensed disturbance voltage. Comparator 40 reproduces the predetermined code transmitted from marker 7 and inputs the code to microprocessor 42. Microprocessor 42 verifies the code with comparison to a programmed internal code list and indicates the comparison product on indicator 9. Resistor 46 provides voltage difference with ground 45.

The surgical implement detector disclosed herein can, of course, be modified in numerous ways without departing from the scope of the invention. For example, an optical scanner and bar code can be added to provide back-up redundancy and/or permit alternative information gathering capacity before or after the surgical procedure, or at locations remote from the wound cavity.

## Claims

1. An apparatus for detecting a surgical implement in human or animal tissue, comprising:
field-generating means (11, 12, 14, 32) for generating an electromagnetic field (20) having a predetermined frequency;
a marker (7) secured to a surgical implement appointed for disposition within said tissue; and
detecting means (5, 8, 10, 16, 42);
**characterised in that**:
said marker (7) has a memory (28) for storing a predetermined code and is arranged to be powered by said electromagnetic field (20) to become operative in the presence of said electromagnetic field to transmit said predetermined code;
said predetermined frequency is in the range of 5 kHz to 3 MHz; and
said detecting means (5, 8, 10, 16, 42) is arranged to receive, decode and verify said predetermined code, and correlate it with said surgical instrument, whereby to enable said surgical instrument to be uniquely identified without the need to physically connect said marker (7) to either said field-generating means (11, 12, 14, 32) or said detecting means (5, 8, 10, 16).

2. An apparatus as claimed in claim 1, wherein said detecting means further comprises indicator means (9) for indicating the reception of said predetermined code.

3. An apparatus as claimed in claim 1 or claim 2, wherein said predetermined frequency ranges from about 50 to 300 kHz.

4. An apparatus as claimed in claim 3, wherein said predetermined frequency is 125 kHz.

5. A method for detecting a surgical implement in human or animal tissue, comprising the steps of:
generating an electromagnetic field (20), having a predetermined frequency in the range of 5 kHz to 3 MHz, in the vicinity of said tissue;
attaching a marker (7) to a surgical instrument appointed for disposition within said tissue, said marker having a memory storing a predetermined code;
powering said marker (7) by placing said marker (7) in the presence of said electromagnetic field (20);
transmitting said predetermined code; and
detecting said transmitted code and correlating it with said surgical implement, thereby enabling said surgical instrument to be uniquely identified.

6. A method as claimed in claim 5, wherein said detecting step further comprises the steps of:
receiving said code;
decoding said code;
verifying said code; and
indicating receipt of said code.

## Patentansprüche

1. Vorrichtung zum Erfassen eines chirurgischen Gerätes in menschlichem oder tierischen Gewebe, umfassend:
ein Felderzeugungsmittel (11, 12, 14, 32) zum Erzeugen eines elektromagnetischen Feldes (20) mit einer vorbestimmten Frequenz;
einen Marker (7), der an einem chirurgischen Gerät befestigt ist, das zur Anordnung in dem Gewebe bestimmt ist, und
ein Erfassungsmittel (5, 8, 10, 16, 42);
**dadurch gekennzeichnet, dass**
der Marker (7) einen Speicher (28) zum Speichern eines vorbestimmten Codes hat und so angeordnet ist, dass er von dem elektromagnetischen Feld (20) geladen wird, so dass er in Gegenwart des elektromagnetischen Feldes (20) betriebsbereit wird, um den vorbestimmten Code zu übertragen;
die vorbestimmte Frequenz im Bereich von 5 kHz bis 3 MHz liegt; und
das Erfassungsmittel (5, 8, 10, 16, 42) so angeordnet ist, dass es den vorbestimmten Code empfängt, decodiert und prüft und ihn mit dem chirurgischen Gerät korreliert, wodurch das chirurgische Gerät eindeutig identifiziert werden kann, ohne den Marker (7) physisch entweder mit dem Felderzeugungsmittel (11, 12, 14, 32) oder dem Erfassungsmittel (5, 8, 10, 16) verbinden zu müssen.

2. Vorrichtung nach Anspruch 1, wobei das Erfassungsmittel des Weiteren Anzeigemittel (9) zum Anzeigen des Empfangs des vorbestimmten Codes umfasst.

3. Vorrichtung nach Anspruch 1 oder Anspruch 2, wobei die vorbestimmte Frequenz im Bereich von etwa 50 bis 300 kHz liegt.

4. Vorrichtung nach Anspruch 3, wobei die vorbestimmte Frequenz 125 kHz ist.

5. Verfahren zum Erfassen eines chirurgischen Gerätes in menschlichem oder tierischen Gewebe, umfassend die folgenden Schritte:
Erzeugen eines elektromagnetischen Feldes (20) mit einer vorbestimmten Frequenz im Bereich von 5 kHz bis 3 MHz in der Nähe des Gewebes;
Befestigen eines Markers (7) an einem chirurgischen Gerät, das zur Anordnung in dem Gewebe bestimmt ist, wobei der Marker einen Speicher hat, der einen vorbestimmten Code speichert;
Laden des Markers (7) durch Anordnen des Markers (7) in Gegenwart des elektromagnetischen Feldes (20);
Senden des vorbestimmten Codes, und
Erfassen des vorbestimmten Codes und Korrelieren desselben mit dem chirurgischen Gerät, wodurch das chirurgische Gerät eindeutig identifiziert werden kann.

6. Verfahren nach Anspruch 5, wobei der Erfassungsschritt des Weiteren folgende Schritte umfasst:
Empfangen des Codes;
Decodieren des Codes;
Überprüfen des Codes; und
Anzeigen des Empfangs des Codes.

## Revendications

1. Dispositif pour détecter un instrument chirurgical dans un tissu humain ou animal comprenant :
des moyens générateurs de champ (11, 12, 14, 32) pour générer un champ électromagnétique (20) ayant une fréquence prédéterminée ;
un marqueur (7) fixé à un instrument chirurgical destiné à être disposé dans ledit tissu ; et des moyens de détection (5, 8, 10, 16, 42) ; **caractérisé en ce que** :
ledit marqueur (7) possède une mémoire (28) pour mémoriser un code prédéterminé et est agencé pour être alimenté par ledit champ électromagnétique (20) pour commencer à fonctionner en présence dudit champ électromagnétique pour transmettre ledit code prédéterminé ;
ladite fréquence prédéterminée est dans la plage de 5 kHz à 3 MHz ; et
lesdits moyens de détection (5, 8, 10, 16, 42) sont agencés pour recevoir, décoder et vérifier ledit code prédéterminé, et le corréler avec ledit instrument chirurgical, de manière à permettre audit instrument chirurgical d'être identifié de façon non-univoque sans qu'il ne soit nécessaire de connecter physiquement ledit marqueur (7) soit auxdits moyens générateurs de champ (11, 12, 14, 32) soit auxdits moyens de détection (5, 8, 10, 16).

2. Dispositif selon la revendication 1, dans lequel lesdits moyens de détection comportent en outre des moyens indicateurs pour indiquer la réception dudit code prédéterminé.

3. Dispositif selon la revendication 1 ou la revendication 2, dans lequel ladite fréquence prédéterminée est comprise entre 50 et 300 kHz.

4. Dispositif selon la revendication 3, dans lequel ladite fréquence prédéterminée est 125 kHz.

5. Procédé pour détecter un instrument chirurgical dans un tissu humain ou animal comprenant les étapes consistant à :
générer un champ électromagnétique (20) ayant une fréquence prédéterminée dans la plage de 5 kHz à 3 MHz au voisinage dudit tissu ;
fixer un marqueur (7) à l'instrument chirurgical destiné à être disposé dans ledit tissu, ledit marqueur ayant une mémoire mémorisant un code prédéterminée ;
alimenter en énergie ledit marqueur (7) en présence dudit champ électromagnétique (20);
transmettre ledit code prédéterminé ; et
détecter ledit code transmis et le corréler avec ledit instrument chirurgical, de manière à permettre audit instrument chirurgical d'être identifié de façon non-univoque.

6. Procédé selon la revendication 5, dans lequel ladite étape de détection comprend en outre les étapes consistant à :
recevoir ledit code ;
décoder ledit code ;
vérifier ledit code ; et
indiquer la réception dudit code.
